(19) Europäisches Patentamt

European Patent Office

Office européen des brevets

(11) **EP 2 803 725 A1**

(12) **EUROPEAN PATENT APPLICATION**

(43) Date of publication:
**19.11.2014 Bulletin 2014/47**

(21) Application number: **13167668.6**

(22) Date of filing: **14.05.2013**

(51) Int Cl.:
*C12N 9/28* (2006.01)          *C11D 3/386* (2006.01)
*C11D 17/04* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **The Procter & Gamble Company
Cincinnati, OH 45202 (US)**

(72) Inventors:
• **JACKSON, Michelle
  Newcastle-upon-Tyne, NE12 9TS (GB)**

• **MAGENNIS, Euan John
  Newcastle-upon-Tyne, NE12 9TS (GB)**

(74) Representative: **Peet, Jillian Wendy
Procter & Gamble Technical Centres Limited
Whitley Road
Longbenton
Newcastle upon Tyne
NE12 9TS (GB)**

(54) **Pouch comprising a cleaning composition**

(57)     A pouch comprising a water-soluble film and a cleaning composition, the cleaning composition being at least partially encompassed within the water-soluble film, wherein the water-soluble film comprises at least 50 % by weight of a water-soluble polyvinyl alcohol (PVOH) resin, the resin having an average viscosity in a range of 10 cP to 30 cP and a degree of hydrolysis in a range of 84% to 98%, and wherein the cleaning composition comprises an alpha-amylase with at least 90% identity with an alpha-amylase selected from the alpha-amylase of SEQ ID NO:1 and the alpha-amylase of SEQ ID NO:2.

FIG. 2

**Description**

FIELD OF THE INVENTION

The present invention relates to a specific amylase with improved cold water properties and an effective way to deliver a cleaning composition comprising said amylase. The cleaning composition is encompassed in a pouch comprising water-soluble films having desired characteristics including good cold water-solubility, wet hand moisture resistance and mechanical properties.

BACKGROUND OF THE INVENTION

Detergent consumer preferences tend towards colder wash temperatures and shorter wash times have resulted in the detergent formulators handling a whole series of different constraints. Consumers also want detergent products which are easy to dose and to manipulate as well as products to use at these lower wash temperatures and shorter wash times, with a similar performance as traditional higher wash temperatures and longer wash cycles; this is an extremely difficult consumer need to meet.
The detergent formulator must greatly improve the efficiency of the detergent ingredients, and of detergent composition as a whole. It is important to maintain good cleaning performance, stain removal performance, good odor profile, and good product stability.
The inventors have discovered that the combination in a pouch of a specific water-soluble film and of a cleaning composition comprising a specific amylase was providing excellent cleaning benefit in short wash time and at cold water temperature.

SUMMARY OF THE INVENTION

The present invention concerns a pouch comprising a water-soluble film and a cleaning composition, the cleaning composition being at least partially encompassed within the water-soluble film. The water-soluble film comprises at least 50 % by weight of a water-soluble polyvinyl alcohol (PVOH) resin, the resin having an average viscosity in a range of 10 cP to 30 cP and a degree of hydrolysis in a range of 84% to 98%. The cleaning composition comprises an alpha-amylase with at least 90% identity with an alpha-amylase selected from the alpha-amylase of SEQ ID NO: 1 and the alpha-amylase of SEQ ID NO:2.
The use of a pouch comprising the water-soluble film of the invention is a particularly effective way to deliver the amylase of the invention in order to maximize the cleaning benefit of the cleaning composition. Also, the pouch form makes the cleaning product easy to use.
Unless specified otherwise, percentages and ratio are expressed in weight.

BRIEF DESCRIPTION OF THE DRAWINGS

The present disclosure can be more readily understood with reference to the appended drawing figures where:

FIG. 1 is a perspective view of a test apparatus used to determine the water disintegration and dissolution times of film samples;
FIG. 2 is a perspective view of the test apparatus and test set-up illustrating the procedure for determining the water-solubility of film samples; and
FIG. 3 is a top view of the test set-up of FIG. 2.

DETAILED DESCRIPTION OF THE INVENTION

THE POUCH

The pouch comprises a water-soluble film and a cleaning composition. The cleaning composition is at least partially encompassed within the water-soluble film. Preferably, the cleaning composition is fully encompassed within the water-soluble film.
The pouch may be a multi-compartment pouch. Each separate compartment of the pouch may comprise a same or different composition. This feature of the disclosure may be utilized to keep compositions containing incompatible ingredients (e.g., chelants and bleach with enzymes) physically separated or partitioned from each other. It is believed that such partitioning may expand the useful life and/or decrease physical instability of such ingredients. Additionally or alternatively, such partitioning may provide aesthetic benefits as described in European Patent Application Number 09161692.0 (filed June 2, 2009 and assigned to the Procter & Gamble Company).

The pouch may be placed in packaging for storage and/or sale. In some embodiments, the package may be a see-through or partially see-through container, for example a transparent or translucent bag, tub, carton or bottle. The package may be made of plastic or any other suitable material, provided the material is strong enough to protect the pouch during transport. The package may comprise 2 or more pouches. This kind of pack is also very useful because the user does not need to open the pack to see how many pouches remain therein. Alternatively, the pack can have non-see-through outer packaging, perhaps with indicia or artwork representing the visually distinctive contents of the pack. In some embodiments, the package may provide at least a partial moisture barrier.

The pouch may be suitable for cleaning applications including, but not limited to cleaning laundry, dishes and the body (e.g. shampoo or soap). The pouch may be suitable for hand and/or machine washing conditions. When machine washing, the pouch may be delivered from a dispensing drawer or may be added directly into the washing machine drum.

The pouch comprises at least one sealed compartment. The sealed compartment comprises the cleaning composition. The pouch may comprise a single compartment or multiple compartments. In embodiments comprising multiple compartments, each compartment may contain identical and/or different compositions. In turn, the compositions may take any suitable form including, but not limited to liquid, solid and combinations thereof (e.g. a solid suspended in a liquid). In some embodiments, the pouch comprises a first, second and third compartment, each of which respectively contains a different first, second and third composition. In some embodiments, the compositions may be visually distinct as described in European Patent Application Number 09161692.0 (filed June 2, 2009 and assigned to the Procter & Gamble Company).

The compartments of multi-compartment pouches may be of the same or different size(s) and/or volume(s). The compartments of the present multi-compartment pouches can be separate or conjoined in any suitable manner. In some embodiments, the second and/or third and/or subsequent compartments are superimposed on the first compartment. In one embodiment, the third compartment may be superimposed on the second compartment, which is in turn superimposed on the first compartment in a sandwich configuration. Alternatively, the second and third compartments may be superimposed on the first compartment. However it is also equally envisaged that the first, second and optionally third and subsequent compartments may be attached to one another in a side by side relationship. The compartments may be packed in a string, each compartment being individually separable by a perforation line. Hence each compartment may be individually torn-off from the remainder of the string by the end-user, for example, so as to pre-treat or post-treat a fabric with a composition from a compartment.

In some embodiments, multi-compartment pouches comprise three compartments consisting of a large first compartment and two smaller compartments. The second and third smaller compartments are superimposed on the first larger compartment. The size and geometry of the compartments are chosen such that this arrangement is achievable. The geometry of the compartments may be the same or different. In some embodiments, the second and optionally third compartment each has a different geometry and shape as compared to the first compartment. In these embodiments, the second and optionally third compartments are arranged in a design on the first compartment. The design may be decorative, educative or illustrative, for example to illustrate a concept or instruction, and/or used to indicate origin of the product. In some embodiments, the first compartment is the largest compartment having two large faces sealed around the perimeter, and the second compartment is smaller, covering less than 75%, or less than 50% of the surface area of one face of the first compartment. In embodiments in which there is a third compartment, the aforementioned structure may be the same but the second and third compartments cover less than 60%, or less than 50%, or less than 45% of the surface area of one face of the first compartment.

The pouch may comprise one or more different films. For example, in single compartment embodiments, the pouch may be made from one wall that is folded onto itself and sealed at the edges, or alternatively, two walls that are sealed together at the edges. In multiple compartment embodiments, the pouch may be made from one or more films such that any given pouch compartment may comprise walls made from a single film or multiple films having differing compositions. In one embodiment, a multi-compartment pouch comprises at least three walls: an outer upper wall; an outer lower wall; and a partitioning wall.

The outer upper wall and the outer lower wall are generally opposing and form the exterior of the pouch. The partitioning wall is interior to the pouch and is secured to the generally opposing outer walls along a seal line. The partitioning wall separates the interior of the multi-compartment pouch into at least a first compartment and a second compartment.

Pouches may be made using any suitable equipment and method. For example, single compartment pouches may be made using vertical form filing, horizontal form filling or rotary drum filling techniques commonly known in the art. Such processes may be either continuous or intermittent. The film may be dampened, and/or heated to increase the malleability thereof.

The method may also involve the use of a vacuum to draw the film into a suitable mold. The vacuum drawing the film into the mold can be applied for 0.2 seconds to 5 seconds, or 0.3 seconds to 3 seconds, or 0.5 seconds to 1.5 seconds, once the film is on the horizontal portion of the surface. This vacuum can be such that it provides an under pressure of between 10 mbar to 1000 mbar, or from 100 mbar to 600 mbar, for example.

The molds, in which the pouches may be made, can have any shape, length, width and depth, depending on the required dimensions of the pouches. The molds may also vary in size and shape from one to another, if desirable. For example, the volume of the final pouches may be 5 ml to 300 ml, or 10 ml to 150 ml, or 20 ml to 100 ml, and that the mold sizes are adjusted accordingly.

Heat can be applied to the film in the process commonly known as thermoforming. The heat may be applied using any suitable means. For example, the film may be heated directly by passing it under a heating element or through hot air, prior to feeding it onto a surface or once on a surface. Alternatively it may be heated indirectly, for example by heating the surface or applying a hot item onto the film. In some embodiments, the film is heated using an infra red light. The film may be heated to a temperature of 50 to 150 °C, 50 to 120 °C, 60 to 130 °C, 70 to 120 °C, or 60 to 90 °C.

Alternatively, the film can be wetted by any suitable means, for example directly by spraying a wetting agent (including water, solutions of the film material or plasticizers for the film material) onto the film, prior to feeding it onto the surface or once on the surface, or indirectly by wetting the surface or by applying a wet item onto the film.

Once a film has been heated and/or wetted, it may be drawn into an appropriate mold, preferably using a vacuum. The filling of the molded film can be accomplished utilizing by any suitable means. In some embodiments, the most preferred method will depend on the product form and required speed of filling. In some embodiments, the molded film is filled by in-line filling techniques. The filled, open pouches are then closed, using a second film, by any suitable method. This may be accomplished while in horizontal position and in continuous, constant motion. The closing may be accomplished by continuously feeding a second film, preferably water-soluble film, over and onto the open pouches and then preferably sealing the first and second film together, typically in the area between the molds and thus between the pouches.

Any suitable method of sealing the pouch and/or the individual compartments thereof may be utilized. Non-limiting examples of such means include heat sealing, solvent welding, solvent or wet sealing, and combinations thereof. Typically, only the area which is to form the seal is treated with heat or solvent. The heat or solvent can be applied by any method, typically on the closing material, and typically only on the areas which are to form the seal. If solvent or wet sealing or welding is used, it may be preferred that heat is also applied. Preferred wet or solvent sealing/ welding methods include applying selectively solvent onto the area between the molds, or on the closing material, by for example, spraying or printing this onto these areas, and then applying pressure onto these areas, to form the seal. Sealing rolls and belts as described above (optionally also providing heat) can be used, for example.

The formed pouches may then be cut by a cutting device. Cutting can be accomplished using any known method. It may be preferred that the cutting is also done in continuous manner, and preferably with constant speed and preferably while in horizontal position. The cutting device can, for example, be a sharp item or a hot item, whereby in the latter case, the hot item 'burns' through the film/ sealing area.

The different compartments of a multi-compartment pouch may be made together in a side-by-side style wherein the resulting, conjoined pouches may or may not be separated by cutting. Alternatively, the compartments can be made separately. In some embodiments, pouches may be made according to a process comprising the steps of:

a) forming a first compartment (as described above);

b) forming a recess within some or all of the closed compartment formed in step (a), to generate a second molded compartment superposed above the first compartment;

c) filling and closing the second compartments by means of a third film;

d) sealing the first, second and third films; and

e) cutting the films to produce a multi-compartment pouch.

The recess formed in step (b) may be achieved by applying a vacuum to the compartment prepared in step a).

In some embodiments, second, and/or third compartment(s) can be made in a separate step and then combined with the first compartment as described in WO 2009/152031 (filed June 13, 2008 and assigned to the Procter & Gamble Company).

In some embodiments, pouches may be made according to a process comprising the steps of:

a) forming a first compartment, optionally using heat and/or vacuum, using a first film on a first forming machine;

b) filling the first compartment with a first composition;

c) on a second forming machine, deforming a second film, optionally using heat and vacuum, to make a second and optionally third molded compartment;

d) filling the second and optionally third compartments;

e) sealing the second and optionally third compartment using a third film;

f) placing the sealed second and optionally third compartments onto the first compartment;

g) sealing the first, second and optionally third compartments; and

h) cutting the films to produce a multi-compartment pouch

The first and second forming machines may be selected based on their suitability to perform the above process. In some embodiments, the first forming machine is preferably a horizontal forming machine, and the second forming machine is preferably a rotary drum forming machine, preferably located above the first forming machine.

It should be understood that by the use of appropriate feed stations, it may be possible to manufacture multi-compartment pouches incorporating a number of different or distinctive compositions and/or different or distinctive liquid, gel or paste compositions.

THE WATER-SOLUBLE FILM

The water-soluble film described herein includes one or more polyvinyl alcohol (PVOH) polymers to make up the PVOH resin content of the film. One or a plurality of PVOH polymers may be selected or blended by the teachings herein to create a film, which is soluble in aqueous solutions. High molecular weight PVOH polymers offer comparatively good residual moisture resistance but are poorly soluble and difficult to thermoform, in part due to thermal sensitivity of the PVOH polymer. Low molecular weight PVOH polymers offer good cold water solubility but are too reactive to residual moisture to function in a commercial or consumer setting, and are difficult to thermoform, in part, due to pinholing and subsequent seepage when filled with liquids or gels. Polyvinyl alcohol is a synthetic resin generally prepared by the alcoholysis, usually termed hydrolysis or saponification, of polyvinyl acetate. Fully hydrolyzed PVOH, where virtually all the acetate groups have been converted to alcohol groups, is a strongly hydrogen-bonded, highly crystalline polymer which dissolves only in hot water - greater than about 60 °C. If a sufficient number of acetate groups are allowed to remain after the hydrolysis of polyvinyl acetate, that is the PVOH polymer is partially hydrolyzed, then the polymer is more weakly hydrogen-bonded, less crystalline, and is generally soluble in cold water - less than 10 °C. As such, the partially hydrolyzed polymer is a vinyl alcohol-vinyl acetate copolymer, that is a PVOH copolymer. Thus, one or more partially hydrolyzed PVOH copolymers are used in the water-soluble film.

The water-soluble film comprises at least 50% by weight of polyvinylalcohol (PVOH) resin comprising one or more PVOH polymers. The water soluble film comprises at least 50% by weight of polyvinylalcohol resin having an average viscosity in a range of 10 cP to 30 cP and a degree of hydrolysis in a range of 84% to 98%.

The water-soluble film may contain a total of at least 55 wt. %, 60 wt. %, 65 wt. %, 70 wt. %, 75 wt. %, 80 wt. %, 85 wt. %, 90 wt. % of PVOH polyvinylalcohol resin. The water-soluble film may comprise at least 60%, or 70%, for example at least 80% or 90% by weight of polyvinylalcohol resin having an average viscosity in a range of 10 cP to 30 cP and/or a degree of hydrolysis in a range of 84% to 98%.

The viscosity of a PVOH polymer ($\mu$) is determined by measuring a freshly made solution using a Brookfield LV type viscometer with UL adapter as described in British Standard EN ISO 15023-2:2006 Annex E Brookfield Test method. It is international practice to state the viscosity of 4% aqueous polyvinyl alcohol solutions at 20 °C. All viscosities specified herein in Centipoise (cP) should be understood to refer to the viscosity of 4% aqueous polyvinyl alcohol solution at 20 °C, unless specified otherwise. Similarly, when a resin is described as having (or not having) a particular viscosity, unless specified otherwise, it is intended that the specified viscosity is the average viscosity for the resin, which inherently has a corresponding molecular weight distribution.

The water-soluble film may comprise at least 50% by weight of polyvinylalcohol resin having an average viscosity of at least 11 cP or 12 cP preferably of at least 13cP or 14 cP, 15 cP, 16 cP, or 17 cP. The water soluble film may comprise at least 50% by weight of polyvinylalcohol resin having an average viscosity of at most 27 cP or 25 cP preferably of at most 20 cP, or 19 cP, or 17.5 cP. The water soluble film may comprise at least 50% by weight of polyvinylalcohol resin having an average viscosity in a range of 12 cP to 25 cP or 13.5 to 20 cP. The water-soluble film may comprises from 0 % to 30 % by weight of a PVOH polymer having an average viscosity less than 11 cP.

The weighted log viscosity average ($\mu$) of the polyvinylalcohol resin of the water-soluble film is calculated as

follow. The $\mu$ is calculated by the formula $\overline{\mu} = e^{\sum W_i \cdot \ln \mu_i}$ where $\mu_1$, is the viscosity for the respective PVOH polymers and $W_i$ is the weight fraction of the respective PVOH polymers.

The PVOH resin may include at least two PVOH polymers, wherein the first PVOH polymer has a viscosity less than the second PVOH polymer. A first PVOH polymer may have a viscosity of at least 8 cP, 10 cP, 12 cP, or 13 cP and at most 40 cP, 20 cP, 15 cP, or 13 cP, for example in a range of 8 cP to 40 cP, or 10 cP to 20 cP, or 10 cP to 15 cP, or 12 cP to 14 cP, or about 13 cP. Furthermore, a second PVOH polymer may have a viscosity of at least 10 cP, 20 cP, or 22 cP and at most 40 cP, 30 cP, 25 cP, or 24 cP, for example in a range of 10 cP to 40 cP, or 20 to 30 cP, or 20 to 25 cP, or 22 to 24, or about 23 cP.

It is well known in the art that the viscosity of PVOH resins is correlated with the weight average molecular weight ($\overline{M}w$) of the PVOH resin, and often the viscosity is used as a proxy for the $\overline{M}w$. Therefore, teachings in the present disclosure regarding the effect of changes in the viscosity of the PVOH resin on the performance or characteristics of the water-soluble films, disclosed herein, correspondingly, apply to the effects of changes in the $\overline{M}w$ of the PVOH resin on the same properties.

The PVOH resin may include a PVOH polymer that has a $\overline{M}w$ in a range of 50,000 to 300,000 Daltons. The PVOH resin may include a first PVOH polymer that has a $\overline{M}w$ in a range of 50,000 to 300,000 Daltons, or 60,000 to 150,000 Daltons; and a second PVOH polymer that has a $\overline{M}w$ in a range of 60,000 to 300,000 Daltons, or 80,000 to 250,000 Daltons.

Depending on the PVOH polymer, the polydispersity index (PDI) of the resin may range from 1.5 to 5, or greater. The PDI of commercial PVOH polymers typically range from 1.8 to 2.3, and typical commercial PVOH polymers may have a PDI of as low as 1.7 and as high as 2.9. The PVOH resin for use herein may have a PDI value of at least 1.3, 1.5, 1.8, 2, 2.5, 3, and at most 6, 5.5, 5, 4.5, 4, 3.5, for example in a range of 1 to 5, or 2 to 4.5, or 2.5 to 4. The PDI value of the PVOH resin can be greater than the PDI value of any individual PVOH polymer included in the resin.

The water-soluble film may comprise at least 50% by weight of polyvinylalcohol resin having a degree of hydrolysis of at least 85% or 87% or 89%. The water-soluble film may comprise at least 50% by weight of polyvinylalcohol resin having a degree of hydrolysis of at most 96%, 94%, 92%, 91 %, or 90%. For example, the water-soluble film comprises at least 50% by weight of polyvinylalcohol resin having a degree of hydrolysis in a range of 84% to 95%, or 85% to 91 %. As used herein, the degree of hydrolysis is expressed as a percentage of vinyl acetate units converted to vinyl alcohol units.

The weight average degree of hydrolysis ($\overline{H^\circ}$) of the polyvinylalcohol resin may be between 80 and 98 %, or between 84 and 96 %, or 87 and 91 %. The $\overline{H^\circ}$ is calculated by the formula $H^\circ = \sum (W_i \cdot H_i)$ where $W_i$ is the weight fraction of the respective PVOH polymers, and $H_i$ is the respective degrees of hydrolysis.

The individual PVOH polymers may have any suitable degree of hydrolysis, as long as the degree of hydrolysis of the total PVOH resin content is within the ranges of the invention.

The water-soluble film may comprise a PVOH resin that has a Resin Selection Index (RSI) in a range of 0.255 to 0.315, or 0.260 to 0.310, or 0.265 to 0.305, or 0.270 to 0.300, or 0.275 to 0.295, preferably 0.270 to 0.300. The RSI is calculated by the formula $\sum (W_i | \mu_i - \mu_i |)/\sum (W_i \mu_i)$, wherein $\mu_i$ is seventeen, $\mu_i$ is the average viscosity each of the respective PVOH polymers, and $W_i$ is the weight fraction of the respective PVOH polymers.

The water-soluble film preferably may be a free-standing film consisting of one layer or a plurality of like layers. The water-soluble-film may be thermoformable. The water-soluble film may further optionally consist essentially of the PVOH resin and the plasticizers and additives as described herein, and be essentially free of other flm layers which would affect solubility, thermoforming performance, or both solubility and thermoforming performance.

The PVOH resin portion of the film may consist essentially of or consist entirely of PVOH polymers. The water-soluble film may also comprise film-forming polymers in addition to PVOH polymers. These additional polymers may be present in the film at a weight percentage of 0.1 to 40%, or at 1 to 30%, based on the total weight of the film. Non-limiting examples include starch, cellulosic materials, sulfopolyesters and mixtures thereof. Further non-limiting examples include: polyalkylene oxides, polyacrylic acid, polyvinyl pyrrolidone, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatin, natural gums such as xanthan, and carrageenans.

The water-soluble film may contain other auxiliary agents and processing agents, such as, but not limited to, plasticizers, lubricants, release agents, fillers, extenders, cross-linking agents, antiblocking agents, antioxidants, detackifying agents, antifoams, nanoparticles such as layered silicate-type nanoclays (e.g., sodium montmorillonite), bleaching agents (e.g., sodium bisulfite or others), and other functional ingredients, in amounts suitable for their intended purpose. Embodiments including plasticizers are preferred. The amount of such agents can be up to 50 wt.%, up to 20 wt%, or up to 15 wt%, or up to 10 wt%, or up to 5 wt.%, e.g., up to 4 wt%, individually or collectively.

The plasticiser may include, but is not limited to, glycerin, diglycerin, sorbitol, ethylene glycol, diethylene glycol, triethylene glycol, tetraethylene glycol, propylene glycol, polyethylene glycols up to 400 MW, neopentyl glycol, trimethylolpropane, polyether polyols, 2-methyl-1,3-propanediol, ethanolamines, and combinations thereof. Preferred plasticizers are glycerin, sorbitol, triethyleneglycol, propylene glycol, 2-methyl-1,3-propanediol, trimethylolpropane, and combinations thereof. The total amount of plasticizer may be in a range of 1 wt.% to 40 wt.%, or 10 wt.% to 40 wt.%, or 15 wt.% to 35 wt.%, or 20 wt.% to 30 wt.%, for example about 25 wt.%. Combinations of glycerin, propylene glycol, and sorbitol may be used. Optionally, glycerin can be used in an amount of 5 wt% to 30 wt%, or 5 wt% to 20 wt%, e.g., 13 wt%. Optionally, propylene glycol may be used in an amount of 1 wt.% to 20 wt.%, or 3 wt.% to 10 wt.%, e.g., about 6 wt.%. Optionally, sorbitol may be used in an amount of 1 wt% to 20 wt%, or 2 wt% to 10 wt%, e.g., 5 wt%.

The water-soluble film can further have a residual moisture content of at least 4 wt.%, for example in a range of 4 to 10 wt. %, as measured by Karl Fischer titration.

Embodiments of the invention include films that have the combined average degree of hydrolysis, weighted log average viscosity, and Resin Selection Index, as presented in the individual cells in Table I below.

TABLE I

|  | $\mu$ **13.5-20** | $\mu$ **14-19** | $\mu$ **15-18** | $\mu$ **16-18** | $\mu$ **17-18** | $\mu$**17.5±0.5** |
|---|---|---|---|---|---|---|
| $\overline{H}°$ **84-90** | $\overline{H}°$ 84-90 $\mu$ 13.5-20 RSI 0.285±0.15 | $\overline{H}°$ 84-90 $\mu$ 14-19 RSI 0.285±0.15 | $\overline{H}°$ 84-90 $\mu$ 15-18 RSI 0.285±0.15 | $\overline{H}°$ 84-90 $\mu$ 16-18 RSI 0.285±0.15 | $\overline{H}°$ 84-90 $\mu$ 17-18 RSI 0.285±0.15 | $\overline{H}°$ 84-90 $\mu$ 17.5±0.5 RSI 0.285±0.15 |
| $\overline{H}°$ **85-89** | $\overline{H}°$ 85-89 $\mu$ 13.5-20 RSI 0.285±0.15 | $\overline{H}°$ 85-89 $\mu$ 14-19 RSI 0.285±0.15 | $\overline{H}°$ 85-89 $\mu$ 15-18 RSI 0.285±0.15 | $\overline{H}°$ 85-89 $\mu$ 16-18 RSI 0.285±0.15 | $\overline{H}°$ 85-89 $\mu$ 17-18 RSI 0.285±0.15 | $\overline{H}°$ 85-89 $\mu$ 17.5±0.5 RSI 0.285±0.15 |
| $\overline{H}°$ **86-88** | $\overline{H}°$ 86-88 $\mu$ 13.5-20 RSI 0.285±0.15 | $\overline{H}°$ 86-88 $\mu$ 14-19 RSI 0-285±0.15 | $\overline{H}°$ 86-88 $\mu$ 15-18 RSI 0.285±0.15 | H" 86-88 $\mu$ 16-18 RSI 0.285±0.15 | $\overline{H}°$ 86-88 $\mu$ 17-18 RSI 0.285±0.15 | $\overline{H}°$ 86-88 $\mu$ 17.5±0.5 RSI 0.285±0.15 |
| $\overline{H}°$ **86.5±0.5** | $\overline{H}°$ 86.5±0.5 $\mu$ 13.5-20 RSI 0.285±0.15 | $\overline{H}°$ 86.5±0.5 $\mu$ 14-19 RSI 0.285±0.15 | $\overline{H}°$ 86.5±0.5 $\mu$ 15-18 RSI 0.285±0.15 | $\overline{H}°$ 86.5±0.5 $\mu$ 16-18 RSI 0.285±0.15 | $\overline{H}°$ 86.5±0.5 $\mu$ 17-18 RSI 0.285±0.15 | $\overline{H}°$ 86.5±0.5 $\mu$ 17.5±0.5 RSI 0.285±0.15 |

The water-soluble film may be formed by, for example, admixing, co-casting, or welding PVOH polymers. If the polymers are first admixed then the water-soluble film is preferably formed by casting the resulting admixture to form a film. If the polymers are welded, the water-soluble film may be formed by, for example, solvent or thermal welding.

The water-soluble film may have any suitable thickness. The water-soluble film may have a thickness between 20 $\mu$m and 125 $\mu$m, for example between 50 $\mu$m and 100 $\mu$m or between 65 $\mu$m and 85 $\mu$m.

The water-soluble film may have a Dissolution Index in a range of 620 to 920, or of 665 to 920, or of 710 to 920. The dissolution Index is measured on a sample of the film having a thickness of 76 $\mu$m.

The Dissolution Index of a film is derived by combining two physical characteristics which are the Dissolution Time and the Burst Strength.

Cold water-solubility is quantified as the Dissolution Time of a film. Dissolution Time is measured using the disclosed Slide Dissolution Test below.

Wet Hand Moisture Resistance is quantified by the sensitivity of a film to moisture and humidity, i.e. the film's wet-handling characteristics. Wet Hand Moisture Resistance is measured using the Burst Strength Test set forth below.

Since each of these parameters relate to different aspects of a consumer's experience, i.e., pouch residue on washed clothing and pouches sticking together due to contact with wet hands, they are weighted differently in the equation utilized to Dissolution Index as defined in equation (1):

$$(1) \quad \text{Dissolution Index} = 7*(\text{Dissolution Time}) + (\text{Burst Strength})$$

Mechanical properties of a film are quantified by its Stress at 100% Elongation and its Ultimate Tensile Strength. These quantities are measured utilizing the ASTM D 882, "Standard Test Method for Tensile Properties of Thin Plastic Sheeting".

These two film mechanical properties are combined to provide the Stress Index of a film as defined by the following equation (2):

$$(2)\ \text{Stress Index} = (\text{Stress at 100\% Elongation}) * (\text{Ultimate Tensile Strength})$$

The water-soluble film may have a Stress Index in a range of 145 to 626, or 155 to 480, or 165 to 325. The Stress Index is measured on a sample of the film having a thickness of 76 $\mu$m.

Without wishing to be bound by theory, it is believed that film having a Dissolution Index that is too high, i.e., above about 920, may provide for a pouch that incompletely dissolves during use. On the other hand, it is believed that a film having a Dissolution Index that is too low, i.e. less than about 620, may provide for a pouch that is too sensitive to moisture and humidity for the consumer market. Furthermore, it is believed that a film having a Stress Index that is too high, i.e., above about 626, may be difficult to process into a pouch due to difficulty in molding into a cavity. On the other hand, it is believed that a film having a Stress Index that is too low, i.e., less than about 145, may be susceptible to pinhole formation during processing into a pouch.

Suitable water-soluble films include M8630, or M8900 which are PVOH copolymer films available from MONOSOL. LLC, Merrillville, IN (USA).

THE CLEANING COMPOSITION

The cleaning compositions may comprise light duty or heavy duty liquid detergent compositions, hard surface cleaning compositions, fabric enhancers, detergent gels commonly used for laundry, bleach and laundry additives, shampoos, body washes, and other personal care compositions.

Cleaning compositions of use in the pouch may take the form of a liquid, solid or a powder. Liquid compositions may comprise a solid. Solids may include powder or agglomerates, such as micro-capsules, beads, noodles or one or more pearlized balls or mixtures thereof. Such a solid element may provide a technical benefit, through the wash or as a pre-treat, delayed or sequential release component; additionally or alternatively, it may provide an aesthetic effect.

The cleaning composition comprises an alpha amylase. The cleaning composition may comprise one or more of the following non-limiting list of ingredients: fabric care benefit agent; detersive enzyme such as lipase, protease, peroxidase, another amylolytic enzyme, e.g., another alpha-amylase, glucoamylase, maltogenic amylase, CGTase, cellulase, mannanase (such as MANNAWAY™ from Novozymes, Denmark), pectinase, pectine lyase, cutinase, laccase, and mixtures thereof; deposition aid; rheology modifier; builder; bleach; bleaching agent; bleach precursor; bleach booster; bleach catalyst; perfume and/or perfume microcapsules (see for example US 5,137,646); perfume loaded zeolite; starch encapsulated accord; polyglycerol esters; whitening agent; pearlescent agent; enzyme stabilizing systems; scavenging agents including fixing agents for anionic dyes, complexing agents for anionic surfactants, and mixtures thereof; optical brighteners or fluorescers; polymer including but not limited to soil release polymer and/or soil suspension polymer; dispersants; antifoam agents; non-aqueous solvent; fatty acid; suds suppressors, e.g., silicone suds suppressors (see: U.S. Publication No. 2003/0060390 A1, ¾65-77); cationic starches (see: US 200410204337 A1 and US 200710219111 A1); scum dispersants (see: US 2003/0126282 A1, ¾89 - 90); dyes; colorants; opacifier; antioxidant; hydrotropes such as toluenesulfonates, cumenesulfonates and naphthalenesulfonates; color speckles; colored beads, spheres or extrudates; clay softening agents. Any one or more of these ingredients is further described in described in European Patent Application Number 09161692.0 (filed June 2, 2009), U.S. Publication Number 2003/0139312A1 (filed May 11, 2000) and U.S. Patent Application Number 61/229981 (filed July 30, 2009), each of which are assigned to the Procter & Gamble Company. Additionally or alternatively, the compositions may comprise surfactants and/or solvent systems, each of which is described below.

Alpha-amylase

The cleaning composition of this invention comprises an alpha-amylase with at least 90%, preferably at least 95%, or at least 98%, or 99% or 100% identity with an alpha-amylase selected from the alpha-amylase of SEQ ID NO:1; and the alpha-amylase of SEQ ID NO:2.

The cleaning composition may comprise at least 0.01% or at least 0.02%, or from 0.05% to 10%, or from 0.1 % to 5% or from 0.2% to 2% of an alpha-amylase.

The alpha-amylase may have 100% identity with the alpha-amylase of SEQ ID NO:1; or SEQ ID NO:2. The alpha-amylase may distinguish from the alpha-amylase of SEQ ID NO:1; or SEQ ID NO:2 by at least 1 mutation, or by at least 2, or 3, or 5, or 10, 15, or 20 mutations. The 1 or more mutation may occur at one or more of the following positions: 2,7, 22, 25, 28, 29, 30, 35, 37,53, 60, 70,72, 75, 83, 87, 91, 93, 108, 116, 125,126, 128, 129, 130, 131, 134, 136, 138, 142, 156, 160, 161, 165, 178, 182, 183, 185, 189, 192, 195, 197, 202, 210, 214, 217, 221, 234, 246, 269, 270, 279, 283, 298, 303, 305, 306,310, 319, 320, 337, 340, 374, 375, 376, 379, 401, 407, 419, 433, 438, 453, 475, 476, and 483.

The alpha-amylase may distinguish from the alpha-amylase of SEQ ID NO:1 or SEQ ID NO:2 by at least 1 mutation, or by at least 2, or 3, or 5, or 10, or 15, or 20, or 30, or 40 mutations at one or more of the following positions: 7, 29, 35, 53, 60, 72, 87, 108, 116, 126, 128, 129, 130, 131, 134, 136, 138, 142, 156, 161, 165, 178, 182, 185, 189, 192, 195, 197, 202, 210, 214, 217, 221, 234, 246,269,303,310,337,340,374,401, and 438.

The alpha-amylase may distinguish from the alpha-amylase of SEQ ID NO:1 or SEQ ID NO:2 by at least 1 mutation, or by at least 2, or 3, or 5, or 10, or 15, or 20, or 30, mutations at one or more of the following positions: 2, 7, 22, 25, 28, 30, 37, 70, 75, 83, 87, 91, 93, 108, 128, 160, 165, 178, 182, 183, 217, 269, 270, 279, 283, 298, 305, 306, 310, 320, 374, 375, 376, 407, 419, 475, and 476.

The alpha-amylase may distinguish from the alpha-amylase of SEQ ID NO:1 or SEQ ID NO:2 by at least 1 mutation, or by at least 2, or 3, or 5, or 10, or 15, or 20 mutations at one or more of the following positions: 83, 125, 128, 131, 160, 178, 182, 183, 185, 189, 279, 305, 319, 320, 379, 407, 433, 453, 475, 476, and 483.

The alpha-amylase may distinguish from the alpha-amylase of SEQ ID NO: 1 or SEQ ID NO:2 by at least 1 mutation, or by at least 2, or 3, or 5 mutations selected from S125A, N128C, T131I, T165I, K178L, T182G, F202Y, S243Q, Y305R, D319T and G475K.

The alpha-amylase may distinguish from the alpha-amylase of SEQ ID NO: 1 or SEQ ID NO:2 by at least a S243Q mutation.

The alpha-amylase may distinguish from the alpha-amylase of SEQ ID NO:1 or SEQ ID NO:2 by at least a S243Q and a G475K mutation.

The alpha-amylase may distinguish from the alpha-amylase of SEQ ID NO:1 or SEQ ID NO:2 by at least a N128C, a K178L, a T182G, a Y305R, and a G475K mutation.

The alpha-amylase may distinguish from the alpha-amylase of SEQ ID NO: 1 or SEQ ID NO:2 by at least a N128C, a K178L, a T182G, a F202Y, a S243Q, a Y305R, a D319T, and a G475K mutation.

The alpha-amylase may distinguish from the alpha-amylase of SEQ ID NO:1) or SEQ ID NO:2 by at least a S125A, a N128C, a K178L, a T182G, a S243Q, a Y305R, and a G475K; mutation.

The alpha-amylase may distinguish from the alpha-amylase of SEQ ID NO:1 or SEQ ID NO:2 by at least a S125A, a N128C, a T131I, a T165I, a K178L, a T182G, a S243Q, a Y305R and a G475K mutation.

Suitable amylases according to the invention can be found in WO2010/115028 and WO2010/115021.

In addition to the alpha-amylase with at least 90%, identity with an alpha-amylase selected from the alpha-amylase of SEQ ID NO:1; and the alpha-amylase of SEQ ID NO:2, the cleaning composition may further comprise an additional amylase. The additional amylase may comprises an amylase with greater than 60% identity to the AA560 alpha amylase endogenous to Bacillus sp. DSM 12649, preferably a variant of the AA560 alpha amylase endogenous to Bacillus sp. DSM 12649 having:

(a) mutations at one or more of positions 9, 26, 149. 182, 186, 202, 257, 295, 299, 323, 339 and 345; and
(b) optionally with one or more, preferably all of the substitutions and/or deletions in the following positions: 118, 183, 184, 195, 320 and 458, which if present preferably comprise R118K, D 183*, G184*, N 195F, R320K and/or R458K.

Suitable commercially available additional amylase enzymes include Stainzyme® Plus, Stainzyme®, Natalase, Termamyl®, Termamyl® Ultra, Liquezyme® SZ (all Novozymes, Bagsvaerd, Denmark) and Spezyme® AA or Ultraphlow (DuPont®, Palo Alto, USA). The additional amylase may be in the form of granulates or liquids or mixtures thereof.

The alpha-amylase of the cleaning composition may comprise at least 10% or 30% or 50% or 70% or 90% by weight of alpha-amylase with at least 90%, identity with an alpha-amylase selected from the alpha-amylase of SEQ ID NO:1; and the alpha-amylase of SEQ ID NO:2.

The alpha-amylase of the cleaning composition may comprise from 10% to 90% or from 30% to 70% of alpha-amylase with at least 90%, identity with an alpha-amylase selected from the alpha-amylase of SEQ ID NO:1; and the alpha-amylase of SEQ ID NO:2.

Surfactants:

The cleaning compositions may comprise surfactant, in particular from 1 % to 80%, or from 5% to 50%, by weight of surfactant.

Surfactants may be of the anionic, nonionic, zwitterionic, ampholytic or cationic type or can comprise compatible mixtures of these types. More preferably surfactants are selected from the group consisting of anionic, nonionic, cationic surfactants and mixtures thereof. Detergent surfactants useful herein are described in U.S. Patent 3,664,961, Norris, issued May 23, 1972, U.S. Patent 3,919,678, Laughlin et al., issued December 30, 1975, U.S. Patent 4,222,905, Cockrell, issued September 16, 1980, and in U.S. Patent 4,239,659, Murphy, issued December 16, 1980. Anionic and nonionic surfactants are preferred.

Useful anionic surfactants can themselves be of several different types. For example, water-soluble salts of the higher fatty acids, i.e., "soaps", are useful anionic surfactants in the compositions herein. This includes alkali metal soaps such as the sodium, potassium, ammonium, and alkyl ammonium salts of higher fatty acids containing from about 8 to about 24 carbon atoms, and preferably from about 12 to about 18 carbon atoms. Soaps can be made by direct saponification of fats and oils or by the neutralization of free fatty acids. Particularly useful are the sodium and potassium salts of the mixtures of fatty acids derived from coconut oil and tallow, i.e., sodium or potassium tallow and coconut soap.

Additional non-soap anionic surfactants which are suitable for use herein include the water-soluble salts, preferably the alkali metal, and ammonium salts, of organic sulfuric reaction products having in their molecular structure an alkyl group containing from about 10 to about 20 carbon atoms and a sulfonic acid or sulfuric acid ester group. (Included in the term "alkyl" is the alkyl portion of acyl groups). Examples of this group of synthetic surfactants include: a) the sodium, potassium and ammonium alkyl sulfates, especially those obtained by sulfating the higher alcohols ($C_8$-$C_{18}$ carbon atoms) such as those produced by reducing the glycerides of tallow or coconut oil; b) the sodium, potassium and ammonium alkyl polyethoxylate sulfates, particularly those in which the alkyl group contains from 10 to 22, preferably from 12 to 18 carbon atoms, and wherein the polyethoxylate chain contains from 1 to 15, preferably 1 to 6 ethoxylate moieties; and c) the sodium and potassium alkylbenzene sulfonates in which the alkyl group contains from about 9 to about 15 carbon atoms, in straight chain or branched chain configuration, e.g., those of the type described in U.S. Patents 2,220,099 and 2,477,383. Especially valuable are linear straight chain alkylbenzene sulfonates in which the average number of carbon atoms in the alkyl group is from about 11 to 13, abbreviated as $C_{11}$-$C_{13}$ LAS.

In some embodiments, the total anionic surfactant, i.e., soap and non-soap anionic, is present in the composition at a weight percentage of 1 wt% to 65 wt%, 2 wt% to 50 wt%, or 5 wt% to 45 wt%.

Preferred nonionic surfactants are those of the formula $R_1(OC_2H_4)_n$ OH, wherein $R_1$ is a $C_{10}$-$C_{16}$ alkyl group or a $C_8$-$C_{12}$ alkyl phenyl group, and n is from 3 to about 80. Particularly preferred are condensation products of $C_{12}$-$C_{15}$ alcohols with from about 5 to about 20 moles of ethylene oxide per mole of alcohol, e.g., $C_{12}$-$C_{13}$ alcohol condensed with about 6.5 moles of ethylene oxide per mole of alcohol.

The Solvent System:

The cleaning composition may comprise a solvent system. The solvent system may contain water, organic solvents and mixture thereof. Preferred organic solvents include 1,2-propanediol, ethanol, glycerol, dipropylene glycol, methyl propane diol and mixtures thereof. The cleaning composition may comprise less than 4% or less than 2% or less than 1 % of water.

Other lower alcohols, $C_1$-$C_4$ alkanolamines such as monoethanolamine and triethanolamine, can also be used. Solvent systems can be absent, for example from anhydrous solid embodiments of the disclosure, but more typically are present at levels in the range of from about 0.1 % to about 98%, preferably at least about 1% to about 50%, more usually from about 5% to about 25%.

Perfume:

The cleaning composition may comprise a perfume. The perfume may comprise a perfume comprising a mixture of at least 5 perfume raw materials and wherein the perfume comprises at least 25 % or 35% or 45% or 55% or 65% or 75% or 85% by weight of perfume raw material selected from: Lavandin Grosso oil; Iso Propyl-2-Methyl Butyrate; Dimethyl cyclohexenyl 3-butenyl ketone; Eucalyptol; Benzyl Acetate; Hexyl Acetate; Methyl Benzoate; 3a,4,5,6,7,7a-hexahydro-4,7- methano-1H-indenyl acetate; Octanal; Cis-3 hexen-1-ol; Nonanal; Ethyl-2-methyl Butyrate; (Z,E)-2,4-dimethyl cyclohex-3-ene-1-carbaldehyde, Tetrahydro-4-methyl-2-(2-methyl propenyl)-2H-pyran; Geraniol; Iso propylbutanal; 2-pentylcyclopentan-1-ol; Dodecenal; d-limonene; Allyl Caproate; Decenal; Tetra Hydro Linalool; (E)-1-trimethyl-1-cyclohex-3(2,6,6-enyl)but-2-en-1-one; 2,4,6- trimethyl-3-cyclohexene-1-carboxaldehyde; Ionone Beta; Prenyl Acetate; 3-(4-tert-butylphenyl)propanal; 1 Carvone; Allyl Cyclohexyl Propionate; Linalool; Phenyl ethyl alcohol; Lemon Oil; Eugenol; Ethyl Vanillin; Cis-3-Hexenyl Acetate; Diphenyl Oxyde; Ionone Alpha; prop-2-enyl 2-cyclohexyloxyacetate; 2-pentyl-Cyclopentanone; Ethyl-2-methyl Pentanoate; [(4Z)-1-cyclooct-4-enyl] methyl carbonate; Cedryl Acetate; Cinnamic Alcohol; 2-methoxyethylbenzene; Phenyl Ethyl Phenyl Acetate; Citronellol; 2-tert-butyl cyclohexyl acetate; Citral; 3alpha,4,5,6,7,7-alpha-hexahydro-4,7-methano-1H-inden-6-yl propanoate; Iso-bornyl iso-butyrate; and mixture thereof.

The cleaning compositions may be prepared by mixing the ingredients together. If a pearlescent material is used it may be added in the late stages of mixing. If a rheology modifier is used, it is preferred to first form a pre-mix within which the rheology modifier is dispersed in a portion of the water and optionally other ingredients eventually used to comprise the compositions. This pre-mix is formed in such a way that it forms a structured liquid. To this structured pre-mix can then be added, while the pre-mix is under agitation, the surfactant(s) and essential laundry adjunct materials, along with water and whatever optional detergent composition adjuncts are to be used.

The pH of the cleaning compositions may be from 4 to 12, from 5 to 11, from 6 to 10, from 6.5 to 8.5, or from 7.0 to 7.5. The cleaning composition may have a pH of from 8 to 10. The cleaning composition may have a pH of from 4 to 8.

The pH of the cleaning composition when liquid is defined as the pH of an aqueous 10% (weight/volume) solution of the detergent at 20°C. For solids and powdered cleaning composition this is defined as the pH of an aqueous 1 % (weight/volume) solution of the detergent at 20°C.

TEST METHODS

SLIDE DISSOLUTION TEST:

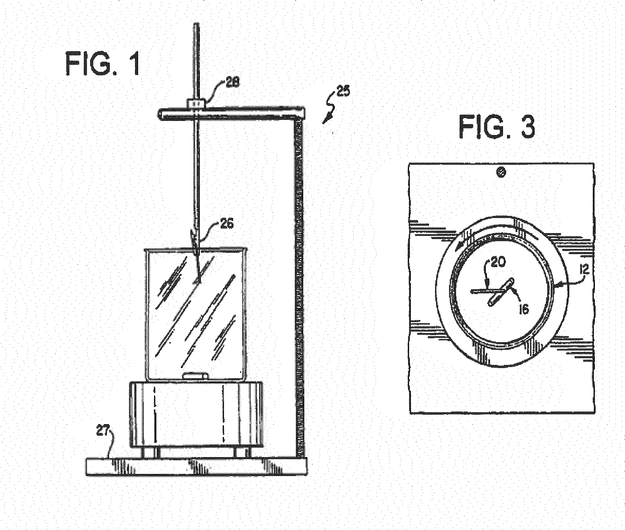

The MONOSOL Test Method 205 (MSTM 205) is disclosed with reference to appended FIGS. 1-3.
Apparatus and Materials:

> 600 mL Beaker 12
> Magnetic Stirrer 14 (Labline Model No. 1250 or equivalent)
> Magnetic Stirring Rod 16 (5 cm)
> Thermometer (0 to 100 °C.,$\pm$1 °C.)
> Template, Stainless Steel (3.8 cm$\times$3.2 cm)
> Timer, (0-300 seconds, accurate to the nearest second)
> Polaroid 35 mm Slide Mount 20 (or equivalent)
> MONOSOL 35 mm Slide Mount Holder 25 (or equivalent, see FIG. 1)
> Distilled Water

Test Specimen:

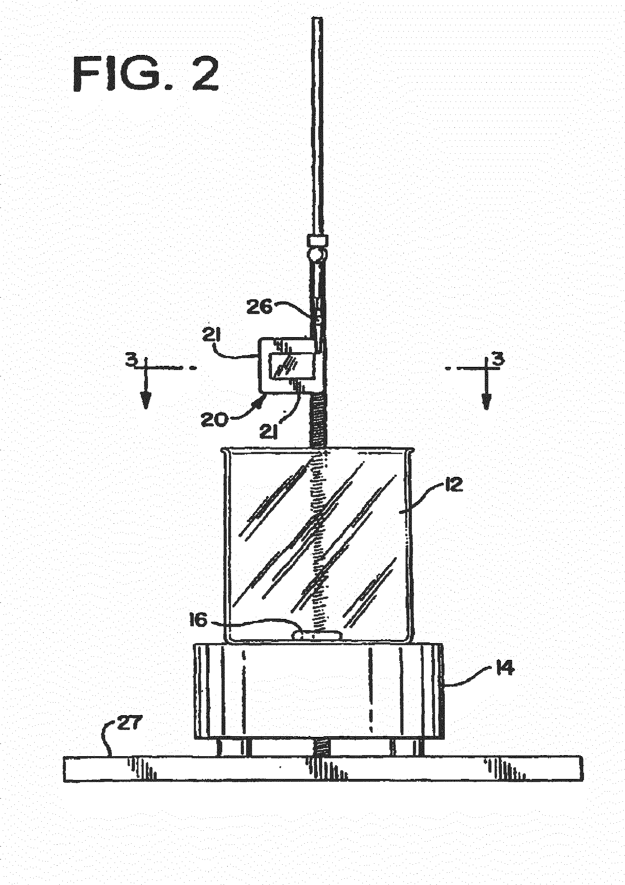

1. Cut three test specimens from film sample using stainless steel template (i.e., 3.8 cm$\times$3.2 cm specimen). If cut from a film web, specimens should be cut from areas of web evenly spaced along the transverse direction of the web.
2. Lock each specimen in a separate 35 mm slide mount 20.
3. Fill beaker 12 with 500 mL of distilled water. Measure water temperature with thermometer and, if necessary, heat or cool water to maintain temperature at 10 °C..
4. Mark height of column of water. Place magnetic stirrer 14 on base 27 of holder 25. Place beaker 12 on magnetic stirrer 14, add magnetic stirring rod 16 to beaker 12, turn on stirrer 14, and adjust stir speed until a vortex develops which is approximately one-fifth the height of the water column. Mark depth of vortex.
5. Secure the 35 mm slide mount 20 in the alligator clamp 26 of the MONOSOL 35 mm slide mount holder 25 (FIG. 1) such that the long end 21 of the slide mount 20 is parallel to the water surface, as illustrated in FIG. 2. The depth adjuster 28 of the holder 25 should be set so that when dropped, the end of the clamp 26 will be 0.6 cm below the surface of the water. One of the short sides 23 of the slide mount 20 should be next to the side of the beaker 12 with the other positioned directly over the center of the stirring rod 16 such that the film surface is perpendicular to the flow of the water, as illustrated in FIG. 3.
6. In one motion, drop the secured slide and clamp into the water and start the timer.
Disintegration occurs when the film breaks apart. When all visible film is released from the slide mount, raise the slide out of the water while continuing to monitor the solution for undissolved film fragments. Dissolution occurs when all film fragments are no longer visible and the solution becomes clear.

Data Recording:

The results should include the following:

> complete sample identification;
> individual and average disintegration and dissolution times; and
> water temperature at which the samples were tested.
> The time for complete dissolution (in seconds) is obtained.

BURST STRENGTH TEST:

A 4 $\mu$l drop of deionized water obtained by reverse osmosis (at 23 °C) is placed on film region to be tested (at about 23 °C and an RH of about 25%) and clamped securely with a 2.5 pounds per square inch ("psig") pressure compressed air behind the film. The droplet is gently placed in the center of the film's clamped circular exposed region which is 21 mm in diameter. The time between droplet placement and burst (i.e., the time at which the pressure is 2.0 psig or lower) is recorded. Film gauge is also recorded. The film region to be tested receives the droplet on its glossy side which forms the exterior surface of a typical pouch. Thus the glossy side of fresh or aged film receives the droplet in the present test.

STRESS AT 100% ELONGATION TEST:

The stress of a film at 100% elongation is measured utilizing the ASTM D 882, "Standard Test Method for Tensile Properties of Thin Plastic Sheeting". The test is conducted on a Model 5544 Instron® Tensile Tester. The Instron® grips utilized in the test may impact the test results. Consequently, the present test is conducted utilizing Instron® grips having model number 2702-032 faces, which are rubber coated and 25 mm wide.

ULTIMATE TENSILE STRENGTH TEST:

The ultimate tensile strength is measured utilizing the ASTM D 882, "Standard Test Method for Tensile Properties of Thin Plastic Sheeting". The test is conducted on a Model 5544 Instron® Tensile Tester. The Instron® grips utilized in the test may impact the test results. Consequently, the present test is conducted utilizing Instron® grips having model number 2702-032 faces, which are rubber coated and 25 mm wide.

EXAMPLES

Examples 1-5 Unit Dose Laundry detergent compositions of the present invention are provided below. Such unit dose formulations can comprise one or multiple compartments. In examples 1-5 the unit dose has one compartment, but similar compositions can be made with two, three, four or five compartments. The film used to encapsulate the cleaning composition is M8630 supplied by MonoSol®,

|  | 1 (wt%) | 2 (wt%) | 3 (wt%) | 4 (wt%) | 5 (wt%) |
|---|---|---|---|---|---|
| Alkylbenzene sulfonic acid | 14.5 | 14.5 | 14.5 | 14.5 | 14.5 |
| $C_{12-18}$ alkyl ethoxy 3 sulfate | 7.5 | 7.5 | 7.5 | 7.5 | 7.5 |
| $C_{12-18}$ alkyl 7-ethoxylate | 13.0 | 13.0 | 13.0 | 13.0 | 13.0 |
| Citric Acid | 0.6 | 0.6 | 0.6 | 0.6 | 0.6 |
| Fatty Acid | 14.8 | 14.8 | 14.8 | 14.8 | 14.8 |
| *Amylase of the invention (mg active) | 6.0 | 12.0 | 8.0 | 2.0 | 10.0 |
| ** Amylase (mg active) | 6.0 |  | 4.0 | 8.0 |  |
| Ethoxylated Polyethylenimine[1] | 4.0 | 4.0 | 4.0 | 4.0 | 4.0 |
| Protease (Purafect Prime®, 40.6 mg active/g) | 1.4 | 2.0 | 0.9 | 1.2 |  |
| Hydroxyethane diphosphonic acid | 1.2 | 1.2 | 1.2 | 1.2 | 1.2 |
| Brightener | 0.3 | 0.3 | 0.3 | 0.3 | 0.3 |
| P-diol | 15.8 | 13.8 | 13.8 | 13.8 | 13.8 |
| Glycerol | 6.1 | 6.1 | 6.1 | 6.1 | 6.1 |
| MEA | 8.0 | 8.0 | 8.0 | 8.0 | 8.0 |
| TIPA |  |  | 2.0 |  |  |
| TEA |  | 2.0 |  |  |  |
| Cumene sulphonate |  |  |  |  | 2.0 |

(continued)

| | 1 (wt%) | 2 (wt%) | 3 (wt%) | 4 (wt%) | 5 (wt%) |
|---|---|---|---|---|---|
| Cyclohexyl dimethanol | | | | 2.0 | |
| Water | 10 | 10 | 10 | 10 | 10 |
| Structurant | 0.14 | 0.14 | 0.14 | 0.14 | 0.14 |
| Perfume | 1.9 | 1.9 | 1.9 | 1.9 | 1.9 |
| Buffers (monoethanolamine) | To pH 8.0 | | | | |
| Solvents (1,2 propanediol, ethanol) | To 100% | | | | |
| *Amylase of the present invention is shown as mgs of active enzyme per 100g of detergent.<br>**Natalase® Plus shown as active enzyme per 100g of detergent.<br>[1]Polyethylenimine (MW = 600) with 20 ethoxylate groups per -NH. | | | | | |

Example 6-8 Multiple Compartment Unit Dose Compositions

Multiple compartment unit dose laundry detergent formulations of the present invention are provided below. In these examples the unit dose has three compartments, but similar compositions can be made with two, four or five compartments. The film used to encapsulate the cleaning composition is M8630 supplied by MonoSol®.

| Base composition 6 | (wt%) |
|---|---|
| Glycerol (min 99) | 5.3 |
| 1,2-propanediol | 10.0 |
| Citric Acid | 0.5 |
| Monoethanolamine | 10.0 |
| Dequest 2010 | 1.1 |
| Potassium sulfite | 0.2 |
| *Amylase of this invention (mg active) | 10.0 |
| Nonionic Marlipal $C_{24}EO_7$ | 20.1 |
| HLAS | 24.6 |
| Optical brightener FWA49 | 0.2 |
| $C_{12-15}$ Fatty acid | 16.4 |
| Polymer Lutensit Z96 | 2.9 |
| Polyethyleneimine ethoxylate PEI600 E20 | 1.1 |
| $MgCl_2$ | 0.2 |
| Solvents (1,2 propanediol, ethanol) | To 100% |

| Composition | 6A | | | | 6B | | |
|---|---|---|---|---|---|---|---|
| Compartment | A | B | C | | A | B | C |
| Volume of each compartment | 40 ml | 5 ml | 5 ml | | 40 ml | 5 ml | 5 ml |
| Active material in Wt.% | | | | | | | |
| Perfume | 1.6 | 1.6 | 1.6 | | 1.6 | 1.6 | 1.6 |
| Dyes | <0.01 | <0.01 | <0.01 | | <0.01 | <0.01 | <0.01 |

(continued)

| Composition | 6A | | | | 6B | | |
|---|---|---|---|---|---|---|---|
| Compartment | A | B | C | | A | B | C |
| TiO2 | 0.1 | | | | | 0.1 | |
| Sodium Sulfite | 0.4 | 0.4 | 0.4 | | 0.3 | 0.3 | 0.3 |
| Acusol 305 | 1.2 | | | | 2 | | |
| Hydrogenated castor oil | 0.14 | 0.14 | 0.14 | | 0.14 | 0.14 | 0.14 |
| **Base Composition 6** | Add to 100% | Add to 100% | Add to 100% | | Add to 100% | Add to 100% | Add to 100% |

*Amylase of the present invention is shown as mgs of active enzyme per 100g of detergent.

| Base composition 2 | (wt%) |
|---|---|
| Glycerol (min 99) | 5.3 |
| 1,2-propanediol | 10.0 |
| Citric Acid | 0.5 |
| Monoethanolamine | 10.0 |
| Dequest 2010 | 1.1 |
| Potassium sulfite | 0.2 |
| *Amylase of this invention (mg active) | 9.0 |
| **Amylase (mg active) | 5.0 |
| Protease (Purafect Prime®, 40.6 mg active/g) | 2.0 |
| Nonionic Marlipal C24EO7 | 20.1 |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

| Composition | 7A | | | | 7B | | |
|---|---|---|---|---|---|---|---|
| Compartment | A | B | C | | A | B | C |
| Volume of each compartment | 40 ml | 5 ml | 5 ml | | 40 ml | 5 ml | 5 ml |
| Active material in Wt.% | | | | | | | |
| Perfume | 1.6 | 1.6 | 1.6 | | 1.6 | 1.6 | 1.6 |
| Dyes | < 0.01 | < 0.01 | < 0.01 | | <0.01 | < 0.01 | < 0.01 |
| TiO2 | 0.1 | - | - | | - | 0.1 | - |
| Sodium Sulfite | 0.4 | 0.4 | 0.4 | | 0.3 | 0.3 | 0.3 |

(continued)

| Composition | 7A | | | | 7B | | |
|---|---|---|---|---|---|---|---|
| Acusol 305 | 1.2 | | | | 2 | - | - |
| Hydrogenated castor oil | 0.14 | 0.14 | 0.14 | | 0.14 | 0.14 | 0.14 |
| **Base Composition 7** | Add to 100% | Add to 100% | Add to 100% | | Add to 100% | Add to 100% | Add to 100% |

*amylase of the present invention is shown as mgs of active enzyme per 100g of detergent.
**Amylase such as Natalase® Plus is shown as active enzyme per 100g of detergent.

| Base composition 8 | (wt%) |
|---|---|
| Glycerol (min 99) | 5.3 |
| 1,2-propanediol | 10.0 |
| Citric Acid | 0.5 |
| Monoethanolamine | 10.0 |
| Dequest 2010 | 1.1 |
| Potassium sulfite | 0.2 |
| Nonionic Marlipal $C_{24}EO_7$ | 20.1 |
| HLAS | 24.6 |
| Optical brightener FWA49 | 0.2 |
| $C_{12-15}$ Fatty acid | 16.4 |
| Polymer Lutensit Z96 | 2.9 |
| Polyethyleneimine ethoxylate PEI600 E20 | 1.1 |
| $MgCl_2$ | 0.2 |
| Solvents (1,2 propanediol, ethanol) | To 100% |

| Composition | 8A | | | | 8B | | |
|---|---|---|---|---|---|---|---|
| Compartment | A | B | C | | A | B | C |
| Volume of each compartment | 40 ml | 5 ml | 5 ml | | 40 ml | 5 ml | 5 ml |
| Active material in Wt.% | | | | | | | |
| Perfume | 1.6 | 1.6 | 1.6 | | 1.6 | 1.6 | 1.6 |
| Protease (Purafect Prime®, 40.6 mg active/g) | | 0.5 | | | | | 2.0 |
| Dyes | <0.01 | <0.01 | <0.01 | | <0.01 | < 0.01 | <0.01 |
| *Amylase of this invention (mg active) | | | 3.0 | | | 3.0 | |
| **Natalase® (mg active) | | | | | | | |
| TiO2 | 0.1 | - | - | | - | 0.1 | - |
| Sodium Sulfite | 0.4 | 0.4 | 0.4 | | 0.3 | 0.3 | 0.3 |
| Acusol 305 | 1.2 | | | | 2 | - | - |
| Hydrogenated castor oil | 0.14 | 0.14 | 0.14 | | 0.14 | 0.14 | 0.14 |

(continued)

| Composition | 8A | | | | 8B | | |
|---|---|---|---|---|---|---|---|
| **Base Composition 8** | Add to 100% | Add to 100% | Add to 100% | | Add to 100% | Add to 100% | Add to 100% |
| *Amylase of the present invention is shown as mgs of active enzyme per 100g of detergent.<br>** active enzyme per 100g of detergent. | | | | | | | |

Examples 9-14

Pouches comprising granular laundry detergent compositions designed for hand washing or top-loading washing machines. The film used to encapsulate the cleaning composition is M8630 supplied by MonoSol®.

| | 9 (wt %) | 10 (wt %) | 11 (wt %) | 12 (wt %) | 13 (wt %) | 14 (wt %) |
|---|---|---|---|---|---|---|
| Linear alkylbenzenesulfonate | 20 | 22 | 20 | 15 | 20 | 20 |
| $C_{12-14}$ Dimethylhydroxyethyl ammonium chloride | 0.7 | 0.2 | 1 | 0.6 | | |
| $AE_3S$ | 0.9 | 1 | 0.9 | | 0.5 | 0.9 |
| $AE_7$ | | | | 1 | 0.0 | 3 |
| Sodium tripolyphosphate | 5 | 0.0 | 4 | 9 | 2 | 0.0 |
| Zeolite A | | 1 | | 1 | 4 | 1 |
| 1.6R Silicate ($SiO_2$:$Na_2O$ at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer[1] | 0.1 | 0.2 | | | | |
| Carboxymethyl cellulose | 1 | 0.3 | 1 | 1 | 1 | 1 |
| Protease (Savinase®, 32.89 mg active/g) | | 0.1 | 0.1 | 0.1 | | 0.1 |
| Lipase - Lipex® (18 mg active /g) | | 0.07 | 0.3 | 0.1 | 0.07 | 0.4 |
| *Amylase of the present invention (mg active) | 0.63 | 1.0 | 2.0 | 0.44 | 0.88 | 0.3 |
| **Amylase (mg active) | | 1.0 | 0.5 | 0.7 | 0.15 | 0.3 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| $MgSO_4$ | 1 | | 1 | 0.5 | 1 | 1 |
| Sodium Percarbonate | | 5.2 | 0.1 | | | |
| Sodium Perborate Monohydrate | 4.4 | | 3.85 | 2.09 | 0.78 | 3.63 |
| NOBS | 1.9 | | 1.66 | | 0.33 | 0.75 |
| TAED | 0.58 | 1.2 | 0.51 | | 0.015 | 0.28 |
| Sulphonated zinc phthalocyanine | 0.0030 | | 0.0012 | 0.0030 | 0.0021 | |
| S-ACMC | 0.1 | | | | 0.06 | |
| Direct Violet 9 | | | 0.0003 | 0.0005 | 0.0003 | |
| Acid Blue 29 | | | | | | 0.0003 |

(continued)

|  | 9 (wt %) | 10 (wt %) | 11 (wt %) | 12 (wt %) | 13 (wt %) | 14 (wt %) |
|---|---|---|---|---|---|---|
| Sulfate/Moisture | Balance | | | | | |

*Amylase of the present invention is shown as mgs of active enzyme per 100g of detergent.
**Amylase such as Stainzyme® Plus is shown as active enzyme per 100g of detergent.

Examples 15-20

Pouches comprising granular laundry detergent compositions designed for front-loading automatic washing machines. The film used to encapsulate the cleaning composition is M8630 supplied by MonoSol®,

|  | 15 (wt%) | 16 (wt%) | 17 (wt%) | 18 (wt%) | 19 (wt%) | 20 (wt%) |
|---|---|---|---|---|---|---|
| Linear alkylbenzenesulfonate | 8 | 7.1 | 7 | 6.5 | 7.5 | 7.5 |
| $AE_3S$ |  | 4.8 |  | 5.2 | 4 | 4 |
| $C_{12-14}$ Alkylsulfate | 1 |  | 1 |  |  |  |
| $AE_7$ | 2.2 |  | 3.2 |  |  |  |
| $C_{10-12}$ Dimethyl hydroxyethylammonium chloride | 0.75 | 0.94 | 0.98 | 0.98 |  |  |
| Crystalline layered silicate ($\delta$-$Na_2Si_2O_5$) | 4.1 |  | 4.8 |  |  |  |
| Zeolite A | 5 |  | 5 |  | 2 | 2 |
| Citric Acid | 3 | 5 | 3 | 4 | 2.5 | 3 |
| Sodium Carbonate | 15 | 20 | 14 | 20 | 23 | 23 |
| Silicate 2R ($SiO_2$:$Na_2O$ at ratio 2:1) | 0.08 |  | 0.11 |  |  |  |
| Soil release agent | 0.75 | 0.72 | 0.71 | 0.72 |  |  |
| Acrylic Acid/Maleic Acid Copolymer | 1.1 | 3.7 | 1.0 | 3.7 | 2.6 | 3.8 |
| Carboxymethylcellulose | 0.15 | 1.4 | 0.2 | 1.4 | 1 | 0.5 |
| Protease - Purafect® (84 mg active/g) | 0.2 | 0.2 | 0.3 | 0.15 | 0.12 | 0.13 |
| Lipase - Lipex® (18.00 mg active/g) |  | 0.15 | 0.1 |  |  |  |
| Cellulase - Celluclean™ (15.6 mg active/g) |  |  |  |  | 0.1 | 0.1 |
| *Amylase of the present invention (mg active) | 4.0 | 2.0 | 1.0 | 0.7 | 6.0 | 3.0 |
| **Amylase (mg active) |  | 2.0 |  | 3.0 |  | 0.5 |
| TAED | 3.6 | 4.0 | 3.6 | 4.0 | 2.2 | I.4 |
| Percarbonate | 13 | 13.2 | 13 | 13.2 | 16 | 14 |
| Na salt of Ethylenediamine-N,N'-disuccinic acid, (S,S) isomer (EDDS) | 0.2 | 0.2 | 0.2 | 0.2 | 0.2 | 0.2 |
| Hydroxyethane di phosphonate (HEDP) | 0.2 | 0.2 | 0.2 | 0.2 | 0.2 | 0.2 |
| $MgSO_4$ | 0.42 | 0.42 | 0.42 | 0.42 | 0.4 | 0.4 |
| Perfume | 0.5 | 0.6 | 0.5 | 0.6 | 0.6 | 0.6 |
| Suds suppressor agglomerate | 0.05 | 0.1 | 0.05 | 0.1 | 0.06 | 0.05 |
| Soap | 0.45 | 0.45 | 0.45 | 0.45 |  |  |
| Sulphonated zinc phthalocyanine (active) | 0.0007 | 0.0012 | 0.0007 |  |  |  |
| S-ACMC | 0.01 | 0.01 |  | 0.01 |  |  |

(continued)

|  | 15 (wt%) | 16 (wt%) | 17 (wt%) | 18 (wt%) | 19 (wt%) | 20 (wt%) |
|---|---|---|---|---|---|---|
| Direct Violet 9 (active) |  |  | 0.0001 | 0.0001 |  |  |
| Sulfate/ Water & Miscellaneous | Balance | | | | | |

*Amylase of the present invention is shown as mgs of active enzyme per 100g of detergent.
**Amylase such as Stainzyme® Plus is shown as active enzyme per 100g of detergent.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A pouch comprising a water-soluble film and a cleaning composition, the cleaning composition being at least partially encompassed within the water-soluble film,
   wherein the water-soluble film comprises at least 50 % by weight of a water-soluble polyvinyl alcohol (PVOH) resin, the resin having an average viscosity in a range of 10 cP to 30 cP and a degree of hydrolysis in a range of 84 % to 98 %, and wherein the cleaning composition comprises an alpha-amylase with at least 90% identity with an alpha-amylase selected from the alpha-amylase of SEQ ID NO:1 and the alpha-amylase of SEQ ID NO:2.

2. The pouch according to any one of the preceding claims, wherein the alpha-amylase has at least 98% identity with an alpha-amylase selected from the alpha-amylase of SEQ ID NO: I and the alpha-amylase of SEQ ID NO:2.

3. A pouch according to claim 1, wherein water-soluble film comprises at least 50 % by weight of a water-soluble polyvinyl alcohol (PVOH) resin, the resin having an average viscosity in a range of 12 cP to 25 cP and a degree of hydrolysis in a range of 85% to 91%.

4. The pouch according to claim 1, wherein the material of the water-soluble film, having any suitable thickness, has a Dissolution Index in a range of 620 to 920 when the film has a thickness of 76 microns, and a Stress Index in a range of 145 to 626, when the film has a thickness of 76 microns.

5. The pouch according to any one of the preceding claims, wherein the water-soluble film comprises from 0 % to 30 % by weight of a PVOH polymer having an average viscosity less than 11 cP.

6. The pouch according to any one of the preceding claims, wherein the PVOH resin comprises a first PVOH polymer having a viscosity in a range of 8 cP to 20 cP; and a second PVOH polymer having a viscosity in a range of about 20 cP to 40 cP.

7. The pouch according to any one of the preceding claims, wherein the water-soluble film further comprising 1 wt.% to 40 wt.% of a plasticizer.

8. The pouch of claim 5, wherein the plasticizer comprises a material selected from the group consisting of glycerin, sorbitol, propylene glycol, 2-methyyl-1,3-propanediol, and a mixture thereof.

9. The pouch according to any one of the preceding claims, wherein the water soluble film has a residual moisture content of 4 wt.% to 10 wt.%.

10. The pouch according to any one of the preceding claims, wherein the water-soluble film having any suitable thickness, is **characterized by** having a Burst Strength of at least 25 seconds, when the film has a thickness of about 76 microns.

11. The pouch according to any one of the preceding claims, wherein the cleaning composition comprises a perfume comprising a mixture of at least 5 perfume raw materials and wherein the perfume comprises at least 25 wt% of perfume raw material selected from: Lavandin Grosso oil; Iso Propyl-2-Methyl Butyrate; Dimethyl cyclohexenyl 3-butenyl ketone; Eucalyptol; Benzyl Acetate; Hexyl Acetate; Methyl Benzoate; 3a,4,5,6,7,7a-hexahydro-4,7-methano-1H-indenyl acetate; Octanal; Cis-3 hexen-1-ol; Nonanal; Ethyl-2-methyl Butyrate; (Z,E)-2,4-dimethyl cyclohex-3-ene-1-carbaldehyde, Tetrahydro-4-methyl-2-(2-methyl propenyl)-2H-pyran; Geraniol; Iso propylbutanal; 2-pentylcyclopentan-1-ol; Dodecenal; d-limonene; Allyl Caproate; Decenal; Tetra Hydro Linalool; (E)-1-trimethyl-1-cyclohex-3(2,6,6-enyl)but-2-en-1-one; 2,4,6- trimethyl-3-cyclohexene-1-carboxaldehyde; Ionone Beta; Prenyl Acetate; 3-(4-tert-butylphenyl)propanal; 1 Carvone; Allyl Cyclohexyl Propionate; Linalool; Phenyl ethyl alcohol; Lemon Oil; Eugenol; Ethyl Vanillin; Cis-3-Hexenyl Acetate; Diphenyl Oxyde; Ionone Alpha; prop-2-enyl 2-cyclohexyloxyacetate; 2-pentyl-Cyclopentanone; Ethyl-2-methyl Pentanoate; [(4Z)-1-cyclooct-4-enyl] methyl carbonate; Cedryl Acetate; Cinnamic Alcohol; 2-methoxyethylbenzene; Phenyl Ethyl Phenyl Acetate; Citronellol; 2-tert-butyl cyclohexyl acetate; Citral; 3alpha,4,5,6,7,7alpha-hexahydro-4,7-methano-1H-inden-6-yl propanoate; Iso-bornyl iso-butyrate; and mixture thereof.

FIG. 3

FIG. 1

FIG. 2

## EUROPEAN SEARCH REPORT

Application Number

EP 13 16 7668

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/094470 A1 (MONOSOL LLC [US]; DENOME FRANK WILLIAM [US]; FRIEDRICH STEVEN G [US];) 4 August 2011 (2011-08-04) * the whole document * | 1-11 | INV.<br>C12N9/28<br>C11D3/386<br>C11D17/04 |
| Y,D | WO 2010/115021 A2 (DANISCO US INC [US]; ESTELL DAVID A [US]; JONES BRIAN E [US]; KOLKMAN) 7 October 2010 (2010-10-07) * the whole document * | 1-11 | |
| Y | WO 2009/061380 A2 (DANISCO US INC GENENCOR DIV [US]; SHAW ANDREW [US]; RAMER SANDRA [US];) 14 May 2009 (2009-05-14) * the whole document * | 1-11 | |
| A | WO 2006/057905 A1 (PROCTER & GAMBLE [US]; KOUVROUKOGLOU STYLIANOS [BE]; DE TROCH LIESBET) 1 June 2006 (2006-06-01) * the whole document * | 1 | |
| A | CHI M C ET AL: "Engineering of a truncated alpha-amylase of Bacillus sp. strain TS-23 for the simultaneous improvement of thermal and oxidative stabilities", JOURNAL OF BIOSCIENCE AND BIOENGINEERING, ELSEVIER, AMSTERDAM, NL, vol. 109, no. 6, 1 June 2010 (2010-06-01), pages 531-538, XP027051714, ISSN: 1389-1723 [retrieved on 2009-12-16] * the whole document * | 1,2 | TECHNICAL FIELDS SEARCHED (IPC)<br><br>C12N<br>C11D |

-/--

The present search report has been drawn up for all claims

| Place of search | Date of completion of the search | Examiner |
|---|---|---|
| The Hague | 26 September 2013 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 13 16 7668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIN L L ET AL: "Impact of Arg210-Ser211 deletion on thermostability of a truncated Bacillus sp. strain TS-23 alpha-amylase", PROCESS BIOCHEMISTRY, ELSEVIER, NL, vol. 43, no. 5, 1 May 2008 (2008-05-01), pages 559-565, XP022588871, ISSN: 1359-5113, DOI: 10.1016/J.PROCBIO.2008.01.020 [retrieved on 2008-02-06] * the whole document * | 1,2 | |
| A | LONG-LIU LIN ET AL: "Production and properties of a raw-starch-degrading amylase from the thermophilic and alkaliphilic Bacillus sp. TS-23", BIOTECHNOLOGY AND APPLIED BIOCHEMISTRY, ACADEMIC PRESS, US, vol. 28, no. 1, 1 January 1998 (1998-01-01), pages 61-68, XP009111923, ISSN: 0885-4513 * the whole document * | 1,2 | |
| A | LIN L-L ET AL: "A gene encoding for an alpha-amylase from thermophilic Bacillus sp. strain TS-23 and its expression in Escherichia coli", JOURNAL OF APPLIED MICROBIOLOGY, OXFORD, GB, vol. 82, no. 3, 1 January 1997 (1997-01-01), pages 325-334, XP002514257, ISSN: 1364-5072, DOI: 10.1046/J.1365-2672.1997.00364.X * the whole document * | 1,2 | TECHNICAL FIELDS SEARCHED (IPC) |

The present search report has been drawn up for all claims

| Place of search | Date of completion of the search | Examiner |
|---|---|---|
| The Hague | 26 September 2013 | van de Kamp, Mart |

EPO FORM 1503 03.82 (P04C01)

## ANNEX TO THE EUROPEAN SEARCH REPORT
## ON EUROPEAN PATENT APPLICATION NO.

EP 13 16 7668

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2013

| Patent document cited in search report | Publication date | Patent family member(s) | | Publication date |
|---|---|---|---|---|
| WO 2011094470 A1 | 04-08-2011 | AR | 080093 A1 | 14-03-2012 |
| | | AR | 080094 A1 | 14-03-2012 |
| | | AU | 2011210549 A1 | 16-08-2012 |
| | | AU | 2011210776 A1 | 09-08-2012 |
| | | CA | 2786735 A1 | 04-08-2011 |
| | | CA | 2786739 A1 | 04-08-2011 |
| | | CA | 2788079 A1 | 04-08-2011 |
| | | CA | 2788152 A1 | 04-08-2011 |
| | | CN | 102725390 A | 10-10-2012 |
| | | CN | 102762612 A | 31-10-2012 |
| | | CN | 102781978 A | 14-11-2012 |
| | | CN | 102858639 A | 02-01-2013 |
| | | EP | 2528822 A1 | 05-12-2012 |
| | | EP | 2528954 A1 | 05-12-2012 |
| | | EP | 2528955 A1 | 05-12-2012 |
| | | EP | 2529002 A1 | 05-12-2012 |
| | | JP | 2013518008 A | 20-05-2013 |
| | | JP | 2013518009 A | 20-05-2013 |
| | | JP | 2013518010 A | 20-05-2013 |
| | | JP | 2013518173 A | 20-05-2013 |
| | | US | 2011186467 A1 | 04-08-2011 |
| | | US | 2011186468 A1 | 04-08-2011 |
| | | US | 2011188784 A1 | 04-08-2011 |
| | | US | 2011189413 A1 | 04-08-2011 |
| | | WO | 2011094470 A1 | 04-08-2011 |
| | | WO | 2011094472 A1 | 04-08-2011 |
| | | WO | 2011094687 A1 | 04-08-2011 |
| | | WO | 2011094690 A1 | 04-08-2011 |
| WO 2010115021 A2 | 07-10-2010 | CA | 2757343 A1 | 07-10-2010 |
| | | CA | 2757347 A1 | 07-10-2010 |
| | | CN | 102378813 A | 14-03-2012 |
| | | CN | 102388132 A | 21-03-2012 |
| | | EP | 2414514 A2 | 08-02-2012 |
| | | EP | 2414515 A2 | 08-02-2012 |
| | | JP | 2012522514 A | 27-09-2012 |
| | | JP | 2012522875 A | 27-09-2012 |
| | | RU | 2011143721 A | 10-05-2013 |
| | | RU | 2011144134 A | 10-05-2013 |
| | | US | 2012045817 A1 | 23-02-2012 |
| | | US | 2012045822 A1 | 23-02-2012 |
| | | WO | 2010115021 A2 | 07-10-2010 |
| | | WO | 2010115028 A2 | 07-10-2010 |
| WO 2009061380 A2 | 14-05-2009 | AU | 2008325250 A1 | 14-05-2009 |
| | | CA | 2704791 A1 | 14-05-2009 |

EPO FORM P0459

For more details about this annex : see Official Journal of the European Patent Office, No. 12/82

**ANNEX TO THE EUROPEAN SEARCH REPORT**
**ON EUROPEAN PATENT APPLICATION NO.**                    EP 13 16 7668

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2013

| Patent document cited in search report | Publication date | Patent family member(s) | Publication date |
|---|---|---|---|
|  |  | CN 101848985 A | 29-09-2010 |
|  |  | EP 2215202 A2 | 11-08-2010 |
|  |  | JP 2011517549 A | 16-06-2011 |
|  |  | KR 20100088675 A | 10-08-2010 |
|  |  | NZ 584434 A | 22-12-2011 |
|  |  | US 2010021587 A1 | 28-01-2010 |
|  |  | WO 2009061380 A2 | 14-05-2009 |
| WO 2006057905 A1 | 01-06-2006 | AT 437215 T | 15-08-2009 |
|  |  | BR PI0517995 A | 21-10-2008 |
|  |  | CA 2586052 A1 | 01-06-2006 |
|  |  | EP 1666579 A1 | 07-06-2006 |
|  |  | ES 2326818 T3 | 20-10-2009 |
|  |  | JP 4672730 B2 | 20-04-2011 |
|  |  | JP 2008518861 A | 05-06-2008 |
|  |  | US 2006276364 A1 | 07-12-2006 |
|  |  | WO 2006057905 A1 | 01-06-2006 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 09161692 A
- WO 2009152031 A
- US 5137646 A
- US 20030060390 A1
- US 200410204337 A1
- US 200710219111 A1
- US 20030126282 A1
- US 20030139312 A1
- US 61229981 A

- WO 2010115028 A
- WO 2010115021 A
- US 3664961 A, Norris
- US 3919678 A, Laughlin
- US 4222905 A, Cockrell
- US 4239659 A, Murphy
- US 2220099 A
- US 2477383 A

SEQUENCE LISTING

<110>   Procter & Gamble

<120>   POUCH COMPRISNG A CLEANING COMPOSITION

<130>   CM3850F

<160>   2

<170>   PatentIn version 3.5

<210>   1
<211>   583
<212>   PRT
<213>   Bacillus sp. TS-23

<400>   1

```
Asn Thr Ala Pro Ile Asn Glu Thr Met Met Gln Tyr Phe Glu Trp Asp
1               5               10              15


Leu Pro Asn Asp Gly Thr Leu Trp Thr Lys Val Lys Asn Glu Ala Ala
        20              25              30


Asn Leu Ser Ser Leu Gly Ile Thr Ala Leu Trp Leu Pro Pro Ala Tyr
        35              40              45


Lys Gly Thr Ser Gln Ser Asp Val Gly Tyr Gly Val Tyr Asp Leu Tyr
        50              55              60


Asp Leu Gly Glu Phe Asn Gln Lys Gly Thr Ile Arg Thr Lys Tyr Gly
65              70              75              80


Thr Lys Thr Gln Tyr Ile Gln Ala Ile Gln Ala Ala Lys Ala Ala Gly
                85              90              95


Met Gln Val Tyr Ala Asp Val Val Phe Asn His Lys Ala Gly Ala Asp
                100             105             110


Gly Thr Glu Phe Val Asp Ala Val Glu Val Asp Pro Ser Asn Arg Asn
            115             120             125


Gln Glu Thr Ser Gly Thr Tyr Gln Ile Gln Ala Trp Thr Lys Phe Asp
        130             135             140


Phe Pro Gly Arg Gly Asn Thr Tyr Ser Ser Phe Lys Trp Arg Trp Tyr
145             150             155             160


His Phe Asp Gly Thr Asp Trp Asp Glu Ser Arg Lys Leu Asn Arg Ile
                165             170             175
```

EP 2 803 725 A1

Tyr Lys Phe Arg Ser Thr Gly Lys Ala Trp Asp Trp Glu Val Asp Thr
    180             185             190

Glu Asn Gly Asn Tyr Asp Tyr Leu Met Phe Ala Asp Leu Asp Met Asp
    195             200             205

His Pro Glu Val Val Thr Glu Leu Lys Asn Trp Gly Thr Trp Tyr Val
210             215             220

Asn Thr Thr Asn Ile Asp Gly Phe Arg Leu Asp Ala Val Lys His Ile
225             230             235             240

Lys Tyr Ser Phe Phe Pro Asp Trp Leu Thr Tyr Val Arg Asn Gln Thr
            245             250             255

Gly Lys Asn Leu Phe Ala Val Gly Glu Phe Trp Ser Tyr Asp Val Asn
            260             265             270

Lys Leu His Asn Tyr Ile Thr Lys Thr Asn Gly Ser Met Ser Leu Phe
            275             280             285

Asp Ala Pro Leu His Asn Asn Phe Tyr Thr Ala Ser Lys Ser Ser Gly
    290             295             300

Tyr Phe Asp Met Arg Tyr Leu Leu Asn Asn Thr Leu Met Lys Asp Gln
305             310             315             320

Pro Ser Leu Ala Val Thr Leu Val Asp Asn His Asp Thr Gln Pro Gly
            325             330             335

Gln Ser Leu Gln Ser Trp Val Glu Pro Trp Phe Lys Pro Leu Ala Tyr
            340             345             350

Ala Phe Ile Leu Thr Arg Gln Glu Gly Tyr Pro Cys Val Phe Tyr Gly
            355             360             365

Asp Tyr Tyr Gly Ile Pro Lys Tyr Asn Ile Pro Gly Leu Lys Ser Lys
    370             375             380

Ile Asp Pro Leu Leu Ile Ala Arg Arg Asp Tyr Ala Tyr Gly Thr Gln
385             390             395             400

Arg Asp Tyr Ile Asp His Gln Asp Ile Ile Gly Trp Thr Arg Glu Gly
            405             410             415

Ile Asp Thr Lys Pro Asn Ser Gly Leu Ala Ala Leu Ile Thr Asp Gly
            420             425             430

```
Pro Gly Gly Ser Lys Trp Met Tyr Val Gly Lys Lys His Ala Gly Lys
        435                 440                 445

Val Phe Tyr Asp Leu Thr Gly Asn Arg Ser Asp Thr Val Thr Ile Asn
        450                 455                 460

Ala Asp Gly Trp Gly Glu Phe Lys Val Asn Gly Gly Ser Val Ser Ile
465                 470                 475                 480

Trp Val Ala Lys Thr Ser Asn Val Thr Phe Thr Val Asn Asn Ala Thr
                485                 490                 495

Thr Thr Ser Gly Gln Asn Val Tyr Val Val Ala Asn Ile Pro Glu Leu
                500                 505                 510

Gly Asn Trp Asn Thr Ala Asn Ala Ile Lys Met Asn Pro Ser Ser Tyr
                515                 520                 525

Pro Thr Trp Lys Ala Thr Ile Ala Leu Pro Gln Gly Lys Ala Ile Glu
        530                 535                 540

Phe Lys Phe Ile Lys Lys Asp Gln Ala Gly Asn Val Ile Trp Glu Ser
545                 550                 555                 560

Thr Ser Asn Arg Thr Tyr Thr Val Pro Phe Ser Ser Thr Gly Ser Tyr
                565                 570                 575

Thr Ala Ser Trp Asn Val Pro
                580
```

```
<210>   2
<211>   484
<212>   PRT
<213>   Bacillus sp. TS-23

<400>   2
```

```
Asn Thr Ala Pro Ile Asn Glu Thr Met Met Gln Tyr Phe Glu Trp Asp
1                   5                   10                  15

Leu Pro Asn Asp Gly Thr Leu Trp Thr Lys Val Lys Asn Glu Ala Ala
                20                  25                  30

Asn Leu Ser Ser Leu Gly Ile Thr Ala Leu Trp Leu Pro Pro Ala Tyr
                35                  40                  45

Lys Gly Thr Ser Gln Ser Asp Val Gly Tyr Gly Val Tyr Asp Leu Tyr
        50                  55                  60
```

Asp Leu Gly Glu Phe Asn Gln Lys Gly Thr Ile Arg Thr Lys Tyr Gly
65              70              75              80

Thr Lys Thr Gln Tyr Ile Gln Ala Ile Gln Ala Ala Lys Ala Ala Gly
            85              90              95

Met Gln Val Tyr Ala Asp Val Val Phe Asn His Lys Ala Gly Ala Asp
            100             105             110

Gly Thr Glu Phe Val Asp Ala Val Glu Val Asp Pro Ser Asn Arg Asn
        115             120             125

Gln Glu Thr Ser Gly Thr Tyr Gln Ile Gln Ala Trp Thr Lys Phe Asp
        130             135             140

Phe Pro Gly Arg Gly Asn Thr Tyr Ser Ser Phe Lys Trp Arg Trp Tyr
145             150             155             160

His Phe Asp Gly Thr Asp Trp Asp Glu Ser Arg Lys Leu Asn Arg Ile
            165             170             175

Tyr Lys Phe Arg Ser Thr Gly Lys Ala Trp Asp Trp Glu Val Asp Thr
            180             185             190

Glu Asn Gly Asn Tyr Asp Tyr Leu Met Phe Ala Asp Leu Asp Met Asp
        195             200             205

His Pro Glu Val Val Thr Glu Leu Lys Asn Trp Gly Thr Trp Tyr Val
    210             215             220

Asn Thr Thr Asn Ile Asp Gly Phe Arg Leu Asp Ala Val Lys His Ile
225             230             235             240

Lys Tyr Ser Phe Phe Pro Asp Trp Leu Thr Tyr Val Arg Asn Gln Thr
            245             250             255

Gly Lys Asn Leu Phe Ala Val Gly Glu Phe Trp Ser Tyr Asp Val Asn
        260             265             270

Lys Leu His Asn Tyr Ile Thr Lys Thr Asn Gly Ser Met Ser Leu Phe
        275             280             285

Asp Ala Pro Leu His Asn Asn Phe Tyr Thr Ala Ser Lys Ser Ser Gly
    290             295             300

Tyr Phe Asp Met Arg Tyr Leu Leu Asn Asn Thr Leu Met Lys Asp Gln
305             310             315             320

```
Pro Ser Leu Ala Val Thr Leu Val Asp Asn His Asp Thr Gln Pro Gly
            325             330             335


Gln Ser Leu Gln Ser Trp Val Glu Pro Trp Phe Lys Pro Leu Ala Tyr
            340             345             350


Ala Phe Ile Leu Thr Arg Gln Glu Gly Tyr Pro Cys Val Phe Tyr Gly
            355             360             365


Asp Tyr Tyr Gly Ile Pro Lys Tyr Asn Ile Pro Gly Leu Lys Ser Lys
    370             375             380


Ile Asp Pro Leu Leu Ile Ala Arg Arg Asp Tyr Ala Tyr Gly Thr Gln
385             390             395             400


Arg Asp Tyr Ile Asp His Gln Asp Ile Ile Gly Trp Thr Arg Glu Gly
            405             410             415


Ile Asp Thr Lys Pro Asn Ser Gly Leu Ala Ala Leu Ile Thr Asp Gly
            420             425             430


Pro Gly Gly Ser Lys Trp Met Tyr Val Gly Lys Lys His Ala Gly Lys
            435             440             445


Val Phe Tyr Asp Leu Thr Gly Asn Arg Ser Asp Thr Val Thr Ile Asn
    450             455             460


Ala Asp Gly Trp Gly Glu Phe Lys Val Asn Gly Gly Ser Val Ser Ile
465             470             475             480


Trp Val Ala Lys
```